# EUROPEAN PATENT SPECIFICATION

(11) **EP 3 197 532 B1**
(45) Date of publication and mention of the grant of the patent: **10.09.2025**
(21) Application number: 15844212.9
(22) Date of filing: 24.09.2015
(51) Int. Cl.: A61M 25/10, A61B 1/012

(54) **COATED BALLOONS AND COATED BALLOON ASSEMBLIES AND RELATED METHODS OF USE AND MANUFACTURE**
BESCHICHTETE BALLONS UND BESCHICHTETE BALLONANORDNUNGEN SOWIE ZUGEHÖRIGE VERFAHREN ZUR VERWENDUNG UND HERSTELLUNG
BALLONNETS REVÊTUS ET ENSEMBLES BALLONNETS REVÊTUS ET PROCÉDÉS ASSOCIÉS D'UTILISATION ET DE FABRICATION

(30) Priority: 25.09.2014 US 201462055384 P
(43) Date of publication of application: 02.08.2017
(60) Divisional application: 25186187.8
(73) Proprietor: Merit Medical Systems, Inc., South Jordan, UT 84095 (US)
(72) Inventor: HOPKINSON, Aaron J., Herriman, Utah 84096 (US); WIERSDORF, Jason Matthew, West Jordan, Utah 84081 (US)
(74) Representative: Cleveland Scott York
(86) International application number: PCT/US2015/051842
(87) International publication number: WO 2016/049262

(56) References cited:
- US-A- 5 512 051
- US-A1- 2002 068 180
- US-A1- 2003 018 353
- US-A1- 2004 148 007
- US-A1- 2007 129 748
- US-A1- 2012 197 380
- US-A1- 2013 006 175

## Description

### TECHNICAL FIELD

This application generally relates to medical devices and assemblies for use in medical procedures, along with related methods. In some embodiments, a medical device may comprise a balloon or other device that is configured to adopt an expanded configuration after having been passed through an elongate channel. The medical device may comprise a lubricious inner coating that facilitates compaction of the medical device when the device is displaced within the elongate channel. The scope of the invention is defined by claims 1-8 relating to a balloon for use in medical procedures, claims 9 and 10 relating to a medical device assembly comprising said balloon, and claims 11 and 12 relating to a method of manufacturing a balloon for deployment.

### BRIEF DESCRIPTION OF THE DRAWINGS

The written disclosure herein describes illustrative embodiments that are non-limiting and non-exhaustive. Reference is made to certain of such illustrative embodiments that are depicted in the figures, in which:
FIG. 1 is a front view of a medical assembly with a balloon in a first compact configuration and disposed distal of a channel of an elongate medical device.
FIG. 2 is a front view of the medical assembly of FIG. 1 with the balloon in an inflated and expanded configuration.
FIG. 3 is a front view of the medical assembly of FIG. 1 with the balloon in a second compact configuration.
FIG. 4 is a front view of the medical assembly of FIG. 1 depicting withdrawal of the balloon into the channel of the elongate medical device after the balloon has been deflated.
FIG. 5 is a cut-away perspective view of a balloon, according to another embodiment, in an inflated configuration.
FIG. 6 is a cross-sectional front view of a balloon body, according to another embodiment, that is being filled with a solution for providing a lubricant layer on an inner surface of the balloon body.
FIG. 7 is a cross-sectional front view of the balloon body of FIG. 6 with a lubricant layer on the inner surface of the balloon body and air being forced through the interior of the balloon body.
FIG. 8 is a column scatter graph depicting the maximum force needed to withdraw various balloon bodies into a particular working channel of an endoscope, where the balloon bodies differ with regard to whether and what type of lubricant layer is disposed on their inner surfaces.
FIG. 9 is a cross-sectional view of an inverted balloon segment where a lubricant layer is disposed on an inner surface of a balloon segment and the inverted balloon is disposed between balloon material that is attached to silicone pads.
FIG. 10 is a cross-sectional view of an inverted balloon segment that lacks lubrication where the balloon segment is disposed between balloon material that is attached to silicone pads.
FIG. 11 is a column scatter graph depicting the test peak friction force of various balloon segments.

### DETAILED DESCRIPTION

It will be readily understood by one of skill in the art having the benefit of this disclosure that the components of the embodiments as generally described and illustrated in the figures herein could be arranged and designed in a wide variety of different configurations. Thus, the following more detailed description of various embodiments, as represented in the figures, is not intended to limit the scope of the present disclosure, but is merely representative of various embodiments. While various aspects of the embodiments are presented in drawings, the drawings are not necessarily drawn to scale unless specifically indicated.

The phrase "coupled to" is used in its ordinary sense, and is broad enough to refer to any suitable coupling or other form of interaction between two or more entities, including mechanical, fluid, and thermal interaction. Two components may be coupled to each other even though they are not in direct contact with each other. For example, two components may be coupled to each other through an intermediate component. The phrase "fluid communication" is used in its ordinary sense, and is broad enough to refer to arrangements in which a fluid (e.g., a gas or a liquid) can flow from one element to another element when the elements are in fluid communication with each other.

The directional terms "proximal" and "distal" are generally used in their ordinary sense. More particularly, when used in connection with a device or component, these terms generally refer to opposite locations on the device or component. The proximal end of a component or device is the end of the device closest to the practitioner when the device is in normal use. The distal end is opposite the proximal end, along the longitudinal axis of the device, or the end farthest from the practitioner during normal use. For example, with regard to elongate devices that are typically disposed within a patient, the proximal end may be the end of the device that sticks out of the patient, while the distal end of the device is the end that is initially inserted into the patient.

An "inner surface" of a balloon or balloon segment refers to the surface on the interior of the balloon when the balloon is in normal operation (or the corresponding surface of a balloon segment). For example, the exposed external surface of an inverted balloon or balloon segment is an inner surface.

The terms "lubricant," "lubricious," etc. are to be given their ordinary meaning as would be understood by one of ordinary skill in the art with the benefit of this disclosure. The term "lubricant layer," as used herein, is specifically defined as a layer comprising a substance (e.g., a solid, liquid, or gel), other than an aqueous solution, that causes a reduction in friction between adjacent surfaces. Although a lubricant layer cannot consist of an aqueous solution, the term "lubricant layer" encompasses, *inter alia,* a layer of a non-aqueous substance that (1) is disposed on an inner surface of a balloon body and (2) decreases friction between adjacent inner surfaces of the balloon body by retaining inflation liquid (e.g., an aqueous liquid) to a greater extent than would the inner surface of the balloon body in the absence of the lubricant layer. For example, a hydrophilic polymer layer that decreases friction by attracting water from an aqueous solution is within the scope of this definition.

The "test peak friction force" for a particular balloon segment is the force measured as set forth in Example 3. An "acute angle," as used herein, is an angle that is 90° or less. Stated differently, as used herein, an acute angle includes a right angle.

In some medical procedures, a balloon or other medical device (e.g., a filter) passes through and emerges distal of an elongate channel, such as a working channel of an endoscope or a channel of a catheter. There, disposed distal of the elongate channel, the balloon or other medical device may be expanded (e.g., inflated in the case of a balloon) for some reason, such as to widen a passageway, secure a stent, collect emboli, etc. The balloon may then be partially or completely deflated. Subsequent to deflation, the practitioner may seek to withdraw the balloon or other medical device from the patient by pulling the balloon or other medical device into and through the elongate channel. However, in some circumstances, withdrawal of the balloon or other medical device is difficult as the balloon or other medical device folds, bunches, deforms, and/or catches in a manner that prevents the balloon or other medical device from transitioning to a sufficiently compact state. In some circumstances, the failure of the balloon or other medical device to transition to a compact state suitable for straightforward withdrawal is due, at least in part, to friction between adjacent portions of an inner surface of a balloon.

In circumstances where a balloon cannot be readily withdrawn into the channel of an elongate medical device (e.g., a working channel of an endoscope), the practitioner may need to withdraw the entire elongate medical device from the patient. Such withdrawal both wastes time and complicates the procedure, as reinsertion and repositioning of an endoscope or other elongate device may be difficult or time-consuming under many circumstances. The prior art document US2002/068180A1 relates to a method of coating the inner surface of the dilatation balloon used with a catheter device with a lubricant in order to overcome friction between surfaces in contact with one another and to consequently reduce the opening pressure required to inflate the balloon.

The balloons and medical device assemblies disclosed herein comprise a lubricant layer comprising polyvinylpyrrolidone that is disposed on an inner surface of a balloon body comprising polyether block amide (PEBAX) or nylon. The lubricant may reduce friction between adjacent portions of the inner surface of the balloon body as the balloon body is withdrawn into an elongate channel. Such reduced friction may facilitate withdrawal of the balloon body into the elongate channel by allowing portions of the balloon body to slide past one other so that the balloon body may adopt a more compact configuration. Further, disposing a lubricant on an inner surface of a balloon body may expand the types of materials from which the balloon body may be manufactured. Stated differently, a lubricant may be disposed on an inner surface of a balloon body that is made of material that would have been unsuitable for use as a balloon body in the absence of the lubricant due to the tendency of the material to stick to itself (and thus impede withdrawal of the balloon into an elongate channel). In other words, the lubricant may allow the balloon body to transition to a compact state needed for withdrawal despite the tendency of the material from which the balloon body is made to stick to itself.

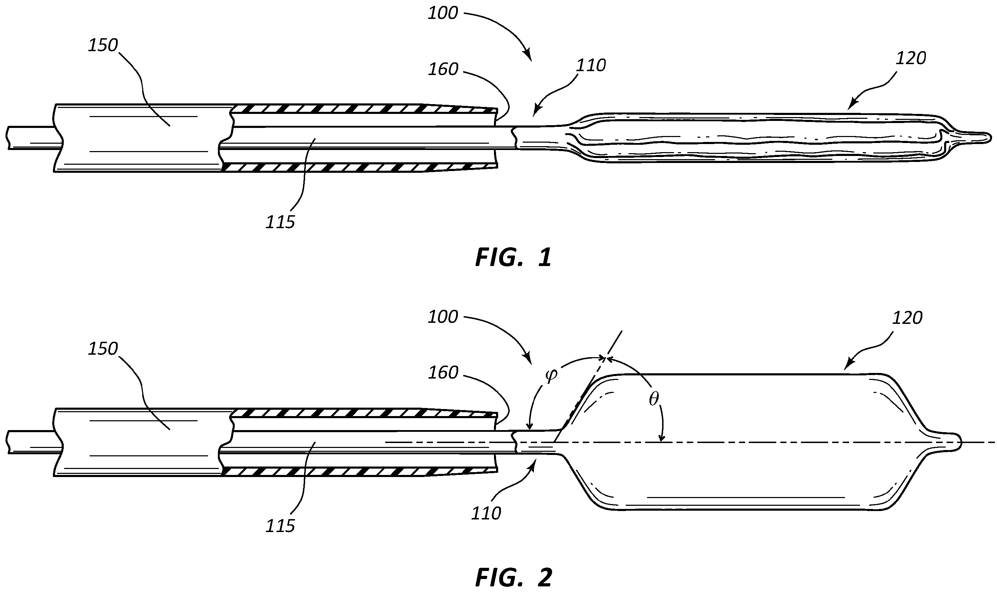

FIG. 1 provides a front view of a medical device assembly 100. The medical device assembly 100 comprises an elongate medical device 150 (such as a catheter or an endoscope) and a balloon catheter 110. The elongate medical device 150 comprises at least one elongate channel 160. In some embodiments, an elongate medical device may comprise other elongate channels as well. For example, in embodiments where the elongate medical device is an endoscope, the elongate medical device may comprise a working channel along with one or more other channels to deliver light and/or transmit an image to the viewer. The balloon catheter 110 may comprise a catheter 115 and a balloon 120 disposed generally distal of the catheter 115. Additionally, the balloon 120 may be coupled to and in fluid communication with the catheter 115.

When the components of the medical device assembly 100 are in the configuration depicted in FIG. 1, a portion of the catheter 115 is disposed within an elongate channel 160 of the elongate medical device 150, while the balloon 120 is disposed distal of the distal end of the elongate medical device 150. The balloon catheter 110 may be disposed in this manner by advancing the balloon catheter 110 through the elongate channel 160 until the balloon 120 is disposed distal of the elongate medical device 150. As the balloon catheter 110 is advanced through the elongate channel 160 of the elongate medical device 150, the balloon 120 may be in a relatively compact and uninflated state, such as that shown in FIG. 1. For example, the balloon may be folded on itself to adopt a compact configuration. In some instances, the balloon 120 may be packaged and supplied to the practitioner in this compact state. Such a relatively compact and uninflated state may allow insertion and advancement of the balloon catheter 110 through the channel 160 without generating substantial resistance, such as due to friction between the outer surface of the balloon 120 and the inner surface of the elongate medical device 150.

FIG. 2 provides a front view of the medical device assembly 100 in a configuration similar to that shown in FIG. 1, but with the balloon 120 in an inflated state. The medical device assembly 100 may be disposed in this manner by forcing fluid into the uninflated balloon 120 (see FIG. 1), thereby causing the balloon 120 to expand and adopt an inflated state (see FIG. 2). In some embodiments, the balloon 120 is inflated by forcing fluid, such as gas and/or liquid, into the balloon 120. More particularly, in some embodiments, the inflation fluid comprises water or an aqueous solution, such as a saline solution.

The balloon 120 may be expanded for any reason. For example, in some embodiments, the balloon 120 is inflated within a lumen of a patient to expand a stent that is disposed within the lumen. More particularly, in some embodiments, an esophageal stent may be deployed in the esophagus of a patient. The balloon 120 may be inserted into the interior of the stent such that inflation of balloon 120 causes the stent to expand radially away from the longitudinal axis of the stent. Such expansion may secure (or more fully secure) the stent within the esophagus. Stents placed at other locations within a patient may be secured in a similar manner.

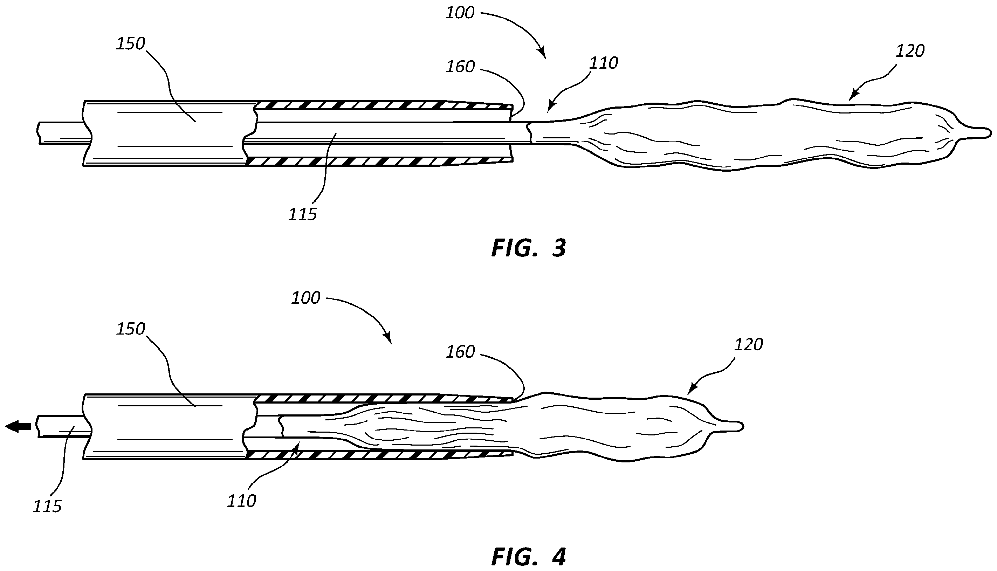

FIG. 3 provides a front view of the medical device assembly 100 in a configuration similar to that shown in FIGS. 1 and 2, except that the balloon 120 is in a deflated state that differs somewhat from the compact uninflated state (e.g., the state of the balloon when supplied from the manufacturer) depicted in FIG. 1. After the balloon 120 has been inflated as shown in FIG. 2, the balloon 120 may be deflated by removing a substantial portion of the fluid that had previously been forced into the balloon 120. When deflated in this manner, the balloon 120 may adopt an irregular configuration, such as that shown in FIG. 3, that differs from the compact state of the balloon 120 when it was advanced through the elongate medical device 150 (see FIG. 1). More particularly, in some embodiments, a balloon 120 in this deflated state may not be as compact as the balloon 120 was when it was advanced through the elongate medical device 150 (compare FIGS. 1 and 3). Stated otherwise, the deflated balloon 120 (see FIG. 3) may have a greater exposed surface area, occupy a larger volume, or define one or more dimensions that are of greater length than the balloon 120 did when it was initially advanced through the elongate medical device 150. Further, the balloon 120 may fold on itself, bunch, deform, or catch on itself in an irregular manner upon deflation.

FIG. 4 provides a front view of the medical assembly 100 in a configuration that differs from the configurations shown in FIGS. 1-3. This configuration shows the withdrawal of the balloon 120 into the channel 160 of the medical device 150 after the balloon 120 has been inflated (see FIG. 2) and deflated (see FIG. 3).

In some embodiments, withdrawal of the balloon 120 into the elongate medical device 150 after the balloon 120 has been inflated and deflated may require a proximal force that is greater than the proximal force that would have been required to withdraw the balloon 120 into the elongate medical device 150 if the balloon 120 were in the same configuration as it was when it was delivered through the elongate channel 160 (e.g., the configuration shown in FIG. 1).

The increase in force needed to withdraw the balloon 120 into the elongate medical device 150 may be caused, at least in part, by the balloon's 120 resistance to transitioning from a less compact configuration to a more compact configuration. Such resistance may be due to one or more factors. For example, upon deflation, the balloon 120 may collapse on itself such that the balloon 120 adopts a relatively non-compact configuration where portions of the inner surface of the balloon 120 contact one another. Such contact may create friction that makes the balloon 120 resistant to transitioning to a more compact configuration as the balloon 120 is withdrawn into the elongate medical device 150. Stated differently, friction arising from the contact of portions of the inner surface of the balloon 120 with each other may prevent portions of the balloon 120 from sliding past one another as needed for the balloon 120 to transition to a shape that is suitable for facile withdrawal of the balloon 120 into the channel 160 of the elongate medical device 150.

Friction between portions of the inner surface of a balloon 120 may be decreased by disposing a lubricant layer on the inner surface of the balloon 120. As the balloon 120 is withdrawn into the channel 160 of the elongate medical device 150, the lubricant layer may decrease the friction between portions of the inner surface of balloon 120, thereby allowing the balloon 120 to more readily transition from a less compact state to a more compact state. In other words, the lubricant layer may decrease the amount of force needed to withdraw the balloon 120 into the channel 160 of the elongate medical device 150 after the balloon 120 has been inflated and deflated relative to an identical balloon that lacks the lubricant layer.

Large balloons may generally be more difficult to withdraw into a particular working channel (e.g., a standard endoscope working channel) than small balloons. For example, a first balloon that has a first inflated diameter (and lacks a lubricant layer) may be more difficult to withdraw into the elongate channel than a second balloon (also lacking a lubricant layer) that has a second inflated diameter that is less than the first inflated diameter. For example, it may be more difficult to withdraw a balloon with an inflated diameter of 10 mm (and lacking a lubricant layer) into the channel of an elongate medical device than to withdraw a similar balloon with an inflated diameter of less than 10 mm into the same channel. Additionally, the difficulty of withdrawing a balloon into a channel may increase as the inflated diameter of the balloon increases. Thus balloons (lacking a lubricant layer) with an inflated diameter of greater than 12, 13, 14, 15, 16, 17 and/or 18 mm may be increasingly difficult to withdraw into an elongate channel than balloons that have smaller inflated diameters. Thus, disposition of a lubricant layer on an inner surface of a relatively large balloon may facilitate this withdrawal.

In addition to inflation diameter, other balloon characteristics may also affect the ability of a balloon to be withdrawn into a channel of an elongate medical device. For example, the shape of a balloon may affect the ability of a balloon to be withdrawn into an elongate channel. Some balloons may be shaped such that a line tangent with the outer surface of the balloon, when the balloon is inflated, intersects the longitudinal axis of the balloon body at a particular acute angle (θ) and a supplementary obtuse angle (φ) (see FIG. 2). Each balloon may have a plurality of tangent lines that intersect with the longitudinal axis of the balloon, each defining a particular acute angle θ. The maximum (i.e., largest) acute angle for each balloon may be referred to as "θ'". In some embodiments, θ' is greater than or equal to 60°, 70°, 75°, 80°, or 85°. The ease of withdrawing a balloon into a channel may be affected by the value of θ'. In other words, embodiments where θ' is relatively large may generally be more difficult to withdraw into an elongate channel than embodiments where θ' is relatively small. Thus, disposing a lubricant layer on the inner surface of balloon bodies where θ' is relatively large may allow these balloon bodies to be withdrawn into an elongate channel in spite of the difficulty of withdrawal that is typically associated with such balloon bodies.

The ease of withdrawing a balloon into a channel may be affected by the size of the channel. For example, balloons withdrawn through relatively small elongate channels may be more likely to become stuck. In some embodiments, balloons are withdrawn into a channel (such as a working channel of endoscope) that has a diameter of less than or equal to 3.8, 2.8, or 2.0 mm. In other or further embodiments, the ratio of the diameter of a balloon to the diameter of the channel through which it is to be withdrawn is greater than 2.5, 4.0, 5.0, and/or 6.0. To facilitate withdrawal of a balloon body into a relatively narrow channel, a lubricant layer may be disposed on an inner surface of the balloon body. Relatedly, disposition of a lubricant on an inner surface of a balloon may also allow for the deployment and withdrawal of a balloon through an elongate device with a relatively small channel. In other words, relative to balloons that lack an inner lubricant layer, balloons with an inner lubricant layer may be deployed through smaller elongate medical devices, which may cause less trauma to a patient upon insertion and withdrawal.

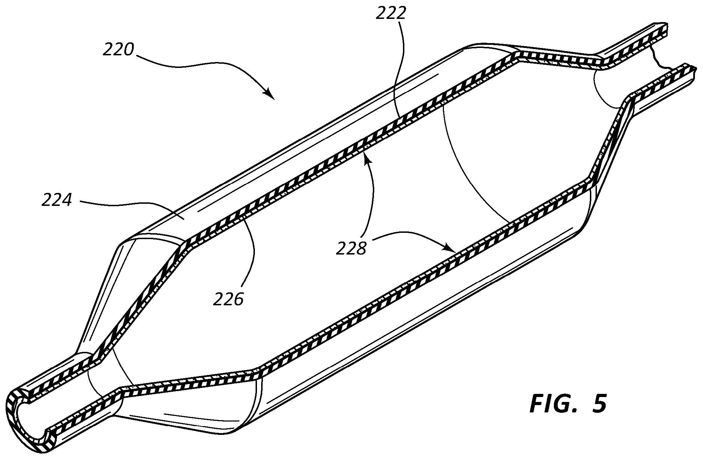

FIG. 5 provides a cut-away perspective view of a balloon 220 that resembles the balloon 120 described above in certain respects. Accordingly, like features are designated with like reference numerals, with the leading digits incremented to "2." Relevant disclosure set forth above regarding similarly identified features thus may not be repeated hereafter. Moreover, specific features of the balloons and other components shown in FIGS. 1-4 may not be shown or identified by a reference numeral in the drawings or specifically discussed in the written description that follows. However, such features may clearly be the same, or substantially the same, as features depicted in other embodiments and/or described with respect to such embodiments. Accordingly, the relevant descriptions of such features apply equally to the features of the balloon 220 depicted in FIG. 5. Any suitable combination of the features, and variations of the same, described with respect to the balloon 120 and related components illustrated in FIGS. 1-4, can be employed with the balloon 220 and related components of FIG. 5, and vice versa. This pattern of disclosure applies equally to further embodiments depicted in subsequent figures and described hereafter, wherein the leading digits may be further incremented.

FIG. 5 provides a cut-away perspective view of a balloon 220 that comprises a balloon body 222 (with an outer surface 224 and an inner surface 226), and a lubricant layer 228 disposed on the inner surface 226 of the balloon body 222.

The balloon body 222 comprises PEBAX or nylon. For example, a first material, such as PEBAX, may define the balloon body 222. The material from which the balloon body 222 is made may have a tendency to stick to itself. In other words, when a first surface of this material is in contact with a second surface of this material, the friction and/or adhesive forces between the contacting materials may prevent or otherwise impede the materials from sliding or slipping past each other. Thus, balloons that are made from such material and lack a lubricant layer may be resistant to changes in configuration that require balloon materials to slide past one another. For example, in instances where balloons have been deflated such that opposing sides of the balloon have come in contact with one another, friction between opposing sides may resist relative movement, causing the sides to "stick" to one another. Resistance to such changes in configuration may be one reason why balloons that lack a lubricant layer on the inner surface of the balloon body may require more force to transition to a compact state suitable for withdrawal through an elongate channel than balloons with a lubricant layer disposed on the inner surface of the balloon body.

The lubricant layer 228 may be used to facilitate the withdrawal of a balloon 220 into a channel. The lubricant layer 228 is hydrophilic and comprises polyvinylpyrrolidone (PVP).

In some embodiments not covered by the claims, where the balloon 220 is inflated by forcing water or an aqueous solution into the balloon 220, an oil lubricant layer, such as silicone, on the inner surface 226 of the balloon body 222 may form droplets in the water, depending on the lubricant and the inflation fluid. In some instances, the presence of droplets may distort or impair the field of view seen through an endoscope, where the object being viewed is on the other side of the balloon 220 (e.g., due to light refraction across the droplets).

In some embodiments not covered by the claims, the oil lubricant and the inflation fluid may be selected to minimize or reduce the amount of refraction that occurs as objects are viewed through the endoscope. For example, the refractive index of the inflation fluid and/or oil may be matched to one other (or to the refractive index of the balloon body), thereby minimizing the amount of distortion that occurs as objects are viewed through the balloon 220.

In the present invention, a hydrophilic coating comprising polyvinylpyrrolidone is deposited on the inner surface 226 of the balloon body 222, thereby functioning as a lubricant layer. A balloon 220 where the balloon body 222 has been coated with a hydrophilic layer in this manner may be inflated by forcing an aqueous solution or mixture into the balloon 220 and subsequently deflated by removing most of the solution or mixture from the balloon 220. The lubricant layer 228 may retain some water that adsorbs to the hydrophilic surface. The retention of water on the inner surface of the hydrophilic coating may decrease the friction between adjacent portions of the hydrophilic coating. In other words, the combination of the hydrophilic coating and the water retained thereon may decrease the friction between portions of the balloon 220 that are brought adjacent to each other as the balloon 220 is deflated.

The use of a hydrophilic coating may reduce or eliminate droplet formation, as the hydrophilic coating is permanently bonded to the inner diameter of the balloon 220.

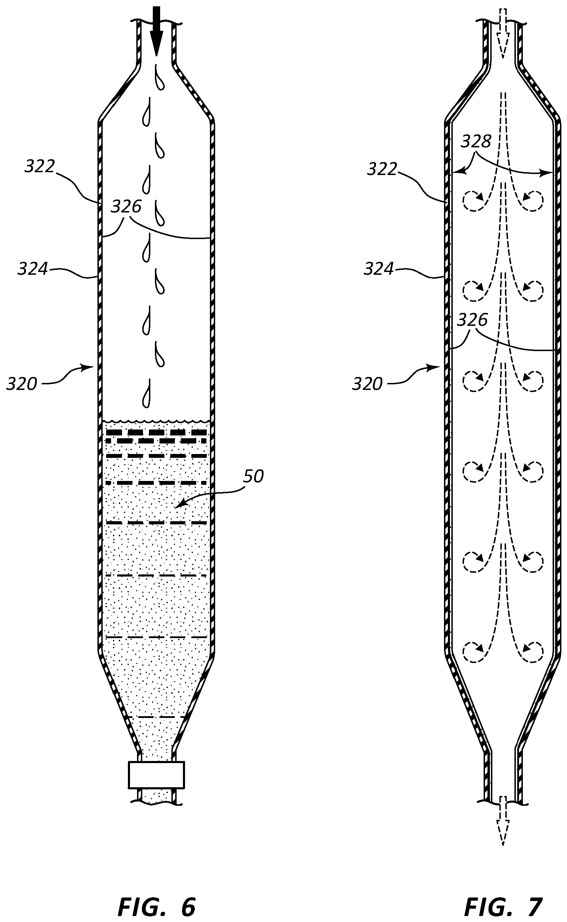

FIG. 6 provides a cross-sectional front view of a balloon 320 that is being filled with a solution 50 for providing a lubricant layer on the inner surface 326 of the balloon body 322. A method of manufacturing a balloon 320 with an inner lubricant layer comprises obtaining a balloon body 322 comprising PEBAX or nylon, applying a polyvinylpyrrolidone coating on an inner surface 326 of the balloon body 322, and curing the coating such that the cured coating provides a bonded, fixed, and/or permanent lubricant layer on the inner surface of the balloon body 322.

In one embodiment, as shown in FIG. 6, a balloon body 322 may be partially or completely filled with a solution 50 comprising the polymer polyvinylpyrrolidone (PVP), water, an alcohol, and a cross-linking catalyst. After the balloon body has been partially or completely filled, the solution 50 may be drained from the balloon body 322 at a controlled rate (e.g., 6.25-12.5 mL/minute). Liquid retained on the inner surface 326 of the balloon body 322 may be dried and/or cured in any suitable manner to create a bonded, fixed, stable, and/or solid inner lubricant layer (i.e., a solid hydrophilic PVP layer). In some embodiments, the drying and/or curing process may comprise one or more of forcing air into the balloon 320, exposing the balloon 320 to UV light, exposing the balloon 320 to heat, and chemically curing the lubricious coating.

More particularly, in some embodiments where UV light is used to partially or completely cure the layer, the balloon 320 may be exposed to UV light by disposing a UV light source outside of the balloon 320. The UV light emitted by the UV light source may pass through the balloon body 322 and promote cross-linking of a lubricant layer (i.e., a PVP layer) disposed on the inner surface 326 of the balloon body 322.

FIG. 7 depicts air being pumped through the balloon of FIG. 6 where most of the solution 50 has been drained from the balloon body 322. As noted above, forcing air through the balloon in this manner may help dry and/or cure the lubricant layer 328.

Lubricant layers may also be used on the interior of devices other than balloons to facilitate their withdrawal into an elongate channel of an elongate medical device. For example, a lubricant layer may be applied to the interior of a filter or basket that has been inserted through an elongate channel. In a manner analogous to that described above in connection with a balloon, the lubricant layer may facilitate compaction of the filter as the filter is withdrawn into the channel. For example, as the filter is withdrawn into an elongate channel of an elongate medical device, the inner lubricant layer may allow interior surfaces of the filter to slide past one another to facilitate compaction and withdrawal of the filter.

Lubricant layers may also be disposed on the inner surface of angioplasty balloons, valvuloplasty balloons, or any other balloon (e.g., balloons used to stabilize passageways for instruments within a patient).

### Example 1-Coating of a balloon with a hydrophilic layer

Balloon bodies of various sizes were filled with an water/isopropyl alcohol solution containing polyvinylpyrrolidone and cross-linking catalyst. After filling each balloon, the solution was drained at a controlled rate between 6.25 and 12.5 mL/minute. After being drained, a layer of solution remained on the inner surface of the balloon, forming a wet interior surface. The balloon body was dried by forcing air through the balloon body and then exposed to UV light (with the lamp outside of the balloon). The resultant balloon had an exterior layer formed from material of the balloon body and a hydrophilic interior layer that included polyvinylpyrrolidone. It was observed that the hydrophilic coating did not substantially diminish the ability of a practitioner to look through the clear balloon to observe objects on the other side of the balloon.

### Example 2

To investigate whether and to what extent a lubricant on the inner surface of a balloon might have on the force needed to withdraw a balloon into a channel after the balloon had been inflated and deflated, three sets of eight 18-19-20 mm × 8 cm multi-stage balloons were obtained. The inner surfaces of the balloons in the first set were coated with a hydrophilic (i.e., PVP) coating as described in Example 1. The second set of balloons was not coated with a lubricant layer. The inner surfaces of the third set of balloons were sprayed with a silicone oil lubricant prior to inflation to form a lubricant layer of silicone oil. The second and third sets of balloons are reference examples.

Each of the 24 balloons was advanced within a 2.8 mm diameter working channel of an endoscope such that each balloon was disposed distal of the distal end of the working channel. The balloons were then inflated to the maximum rated pressure for 30 seconds and then deflated by vacuum. With the balloon catheter still under vacuum and the working channel in a straight orientation, a proximal force was applied to pull the balloon into the working channel of the endoscope at a rate of 500 mm per minute. The magnitude of the force was monitored, and the maximum force needed to pull the balloon into the working channel of the endoscope (i.e., the maximum withdraw load) was recorded. The average maximum withdraw load for each set of balloon catheters was also calculated.

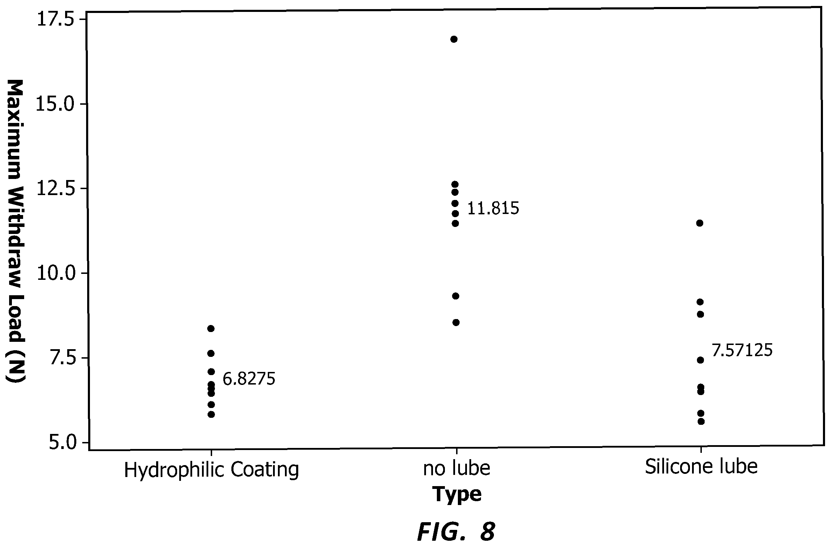

The results are shown in FIG. 8, which is a column scatter graph depicting the maximum force needed to withdraw the balloons into the working channel of the endoscope. The graph shows that the force needed to withdraw a balloon with no lubricant layer on the inner surface (see the center column of graph) is generally higher than the force needed to withdraw a balloon which had either a silicone lube inner layer or a hydrophilic coating disposed on the inner surface.

The average force needed to withdraw balloons that had been coated with a hydrophilic layer into the channel of the working endoscope was 6.8 Newtons (N). The average force needed to withdraw balloons that had been lubricated by spraying silicone on an inner surface was 7.6 N. The average force needed to withdraw multi-stage balloons that lacked an inner lubricant layer into the channel of the endoscope was 11.8 N.

### Example 3

To investigate the degree to which various coatings decrease friction between adjacent inner surfaces of a balloon body, the "test peak friction force" for various balloon materials was measured as described below.

First, three sets of 7033 SA01 PEBAX balloon bodies were obtained. The inner surfaces of the balloon bodies in the first set were coated with the hydrophilic PVP coating as described above in Example 1. The second set of balloons was not coated with a lubricant layer. The third set of balloons was coated with a hydrophobic layer by spraying silicone oil with a nominal viscosity of 100 mPa·S on the inner surface of the balloons. The second and third sets of balloons are reference examples.

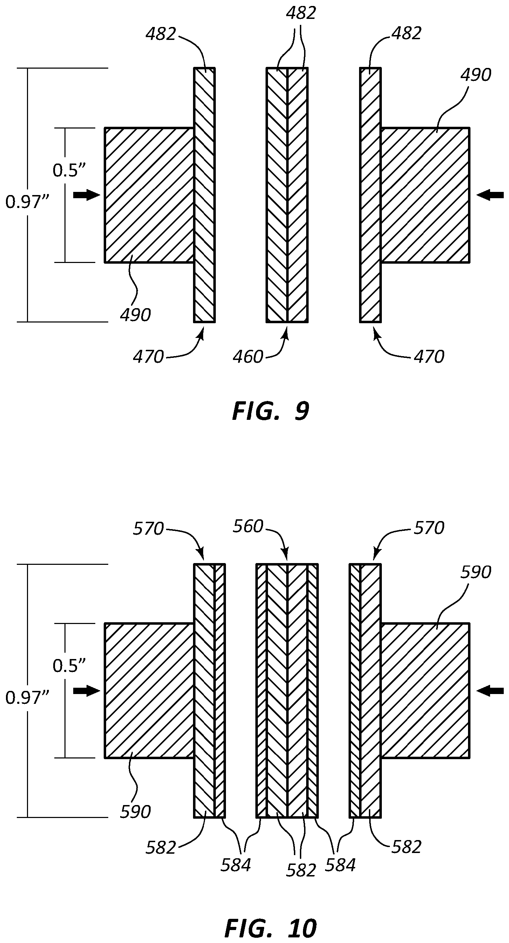

Each of the balloons was then inverted (thereby exposing the inner surface of the balloon), cut into lengths of approximately 2.46 cm (0.97 inches), and flattened. FIG. 9 depicts the setup used to measure the test peak friction force of a balloon segment 460 that lacks a lubricant layer (e.g., a balloon segment of a balloon defined by a first material 482). FIG. 10 depicts the setup for measuring the test peak friction force of balloon segments 560 with a first material 582 and a second material 584 disposed on the inner surface of the first material 582.

As depicted in FIG. 9, to ascertain the test peak friction force for a balloon segment 460 that lacks a lubricant layer, a flattened balloon segment 460 was sandwiched between a sheet 470 of balloon material 482 that was identical with the material of the balloon segment 460 while the entire testing system was submerged in a saline bath (37 °C). The sheets 470 of balloon material 482 were attached to two 80A durometer silicone pads 490. With these components disposed in this manner, the sandwich structure was compressed by moving the silicone pads 490 toward each other to provide a normal force (100 gram-force; 0.98 × 10⁻³ N) to the inverted balloon segment 460. The inverted balloon segment 460 was then pulled upward at a rate of 1 cm/s for 1 cm, and the maximum force required to maintain this pull rate (i.e., the test peak friction force) was recorded by a force gauge coupled to the inverted balloon segment 460.

The test peak friction forces for balloon segments 560 that had been coated with either silicone oil or PVP were measured according to an analogous procedure. As depicted in FIG. 10, the inverted balloon segment 560 (comprising a first material 582 and a second material 584 (silicone oil or PVP) disposed on the inner surface of the first material 582) was disposed between two sheets 570 that each comprise a first material 582 and a second material 584 disposed on the inner surface of the first material 582. For each sheet 570, the second material 584 is oriented toward the inverted balloon segment 560. The test peak friction force was then measured as described above in connection with the balloon segment 460 that lacks a lubricant layer by compressing the sandwich structure and pulling the inverted balloon segment 560 in an upward direction at a constant rate.

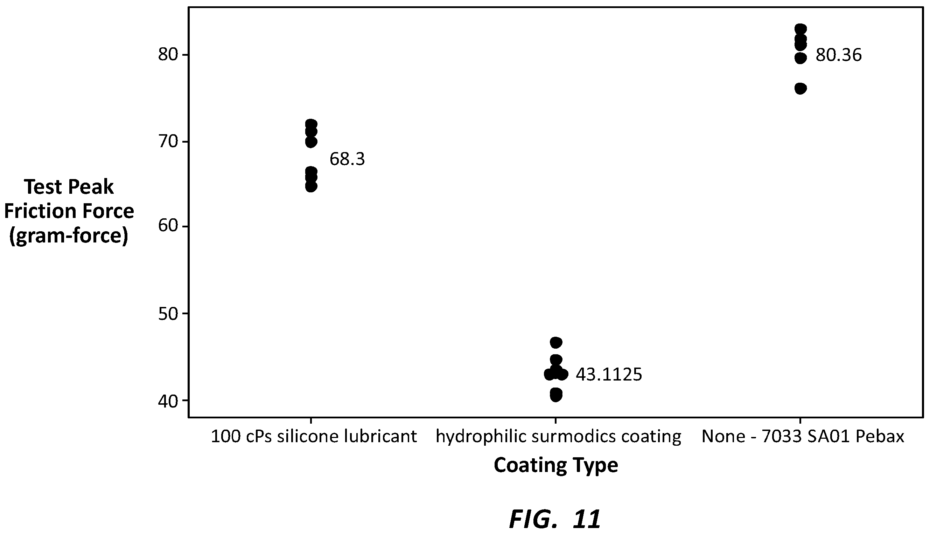

The results of these experiments are shown in FIG. 11, which is a column scatter graph depicting the test peak friction force for each balloon segment. On average, the test peak friction force for balloon segments that had been coated with a hydrophobic (i.e., silicone oil) or hydrophilic (i.e., PVP) layer was lower than the test peak friction force for balloon segments that had lacked a lubricant layer. More particularly, the average test peak friction force for balloon segments that had been lubricated with silicone oil spray was 68.3 gram-force, a 15% reduction in test peak friction force relative to the average test peak friction force for a balloon segment that had lacked a lubricant layer (80.4 gram-force). The average test peak friction force for balloon segments that had been coated with a hydrophilic PVP layer was 43.1 gram-force, a 46% reduction in test peak friction force relative to the average test peak friction force for a balloon segment that had lacked a lubricant layer (80.4 gram-force). In other words, the test peak friction force for balloon segments having a second material disposed on the inner surface of a first material may be at least 10%, 15%, 25%, 35%, or 45% lower than the test peak friction force for balloon segments of only first material. Further, the data shown in FIG. 11 demonstrate that the test peak friction force for a balloon segment comprising a first material with a second material disposed on the inner surface of the first material may be less than 75, 70, 65, 60, 55, 50, and/or 45 gram-force.

## Claims

1. A balloon for use in medical procedures, the balloon comprising:
a first material defining a balloon body configured for delivery through a channel of an elongate medical device, the first material comprising polyether block amide (PEBAX) or nylon, wherein the balloon body comprises a diameter, when inflated, of 10 mm or greater; and
a second material disposed on an inner surface of the balloon body, the second material comprising a dried, solid lubricant layer that is hydrophilic and comprises polyvinylpyrrolidone, wherein the lubricant layer is cured and permanently bonded to the inner surface of the balloon body; and
wherein the lubricant layer is configured to decrease the amount of force needed to withdraw the balloon body into the channel of the elongate medical device after the balloon body has been inflated and deflated relative to an identical balloon that lacks the lubricant layer.

2. The balloon for use of claim 1, wherein the lubricant layer is configured to facilitate compaction of the balloon body as the balloon body is withdrawn into a channel of an elongate medical device.

3. The balloon for use of claim 1, wherein the lubricant layer is configured to retain water after being inflated with an aqueous solution.

4. The balloon for use of any one of claims 1-3, wherein the balloon body comprises nylon.

5. The balloon for use of any one of claims 1-3, wherein the balloon body comprises polyether block amide (PEBAX).

6. The balloon for use of any one of claims 1-5, wherein the balloon body, when inflated, comprises an exterior surface such that a line tangent with the exterior surface intersects a longitudinal axis of the balloon body at an acute angle that is greater than 65 degrees.

7. The balloon for use of any one of claims 1-6, wherein the balloon is configured to expand an esophageal stent.

8. The balloon for use of any one of claims 1-7, wherein the lubricant comprises a layer of polyvinylpyrrolidone that has been cured by a process comprising exposure to UV light.

9. A medical device assembly comprising:
a balloon for use according to any of claims 1-8 comprising a balloon body and a lubricant layer; and
an elongate medical device comprising an elongate channel;
wherein the balloon body is configured for delivery through the elongate channel of the elongate medical device, and the lubricant layer decreases the amount of force needed to withdraw the balloon body into the elongate channel of the elongate medical device after the balloon body has been both inflated and deflated relative to an identical balloon that lacks the lubricant layer.

10. The assembly of claim 9, wherein the balloon comprises a diameter of greater than 12 mm when inflated and the elongate channel of the elongate medical device comprises a diameter of 3.8 mm or less, and wherein the ratio of the diameter of the balloon when inflated to the diameter of the elongate channel is greater than 4.

11. A method of manufacturing a balloon for deployment within a cavity of patient, the method comprising:
obtaining a balloon body comprising polyether block amide (PEBAX) or nylon;
applying a polyvinylpyrrolidone coating on an inner surface of the balloon body; and curing the coating;
wherein the cured coating provides a permanent lubricant on the inner surface of the balloon body.

12. The method of claim 11, wherein applying the coating comprises at least partially filling the balloon body with a mixture comprising polyvinylpyrrolidone and a catalyst and wherein curing the coating comprises exposing the coating to UV light.

## Patentansprüche

1. Ballon zur Verwendung in medizinischen Prozeduren, wobei der Ballon Folgendes umfasst:
ein erstes Material, das einen Ballonkörper definiert, der zur Abgabe durch einen Kanal einer länglichen medizinischen Vorrichtung konfiguriert ist, wobei das erste Material Polyetherblockamid (PEBAX) oder Nylon umfasst, wobei der Ballonkörper im aufgeblasenen Zustand einen Durchmesser von 10 mm oder mehr umfasst; und
ein zweites Material, das auf einer inneren Oberfläche des Ballonkörpers angeordnet ist, wobei das zweite Material eine getrocknete, feste Gleitmittelschicht umfasst, die hydrophil ist und Polyvinylpyrrolidon umfasst, wobei die Gleitmittelschicht gehärtet und dauerhaft mit der inneren Oberfläche des Ballonkörpers verbunden ist; und
wobei die Gleitschicht konfiguriert ist, um den Kraftaufwand zu verringern, der erforderlich ist, um den Ballonkörper in den Kanal der länglichen medizinischen Vorrichtung zurückzuziehen, nachdem der Ballonkörper aufgeblasen und entleert wurde, im Vergleich zu einem identischen Ballon, dem die Gleitschicht fehlt.

2. Ballon zur Verwendung nach Anspruch 1, wobei die Gleitmittelschicht konfiguriert ist, um die Verdichtung des Ballonkörpers zu erleichtern, wenn der Ballonkörper in einen Kanal einer länglichen medizinischen Vorrichtung zurückgezogen wird.

3. Ballon zur Verwendung nach Anspruch 1, wobei die Gleitmittelschicht konfiguriert ist, um Wasser zurückzuhalten, nachdem sie mit einer wässrigen Lösung aufgeblasen wurde.

4. Ballon zur Verwendung nach einem der Ansprüche 1 bis 3, wobei der Ballonkörper Nylon umfasst.

5. Ballon zur Verwendung nach einem der Ansprüche 1 bis 3, wobei der Ballonkörper Polyetherblockamid (PEBAX) umfasst.

6. Ballon zur Verwendung nach einem der Ansprüche 1 bis 5, wobei der Ballonkörper im aufgeblasenen Zustand eine äußere Oberfläche umfasst, so dass eine Linie, welche die äußere Oberfläche tangiert, eine Längsachse des Ballonkörpers in einem spitzen Winkel schneidet, der größer als 65 Grad ist.

7. Ballon zur Verwendung nach einem der Ansprüche 1 bis 6, wobei der Ballon konfiguriert ist, um einen Ösophagus-Stent zu expandieren.

8. Ballon zur Verwendung nach einem der Ansprüche 1 bis 7, wobei das Gleitmittel eine Schicht aus Polyvinylpyrrolidon umfasst, die durch einen Prozess ausgehärtet wurde, der die Bestrahlung mit UV-Licht umfasst.

9. Medizinische Vorrichtungsanordnung, umfassend:
einen Ballon zur Verwendung nach einem der Ansprüche 1 bis 8, umfassend einen Ballonkörper und eine Gleitmittelschicht;
und
eine längliche medizinische Vorrichtung, umfassend einen länglichen Kanal;
wobei der Ballonkörper zur Abgabe durch den länglichen Kanal der länglichen medizinischen Vorrichtung konfiguriert ist, und die Gleitmittelschicht den Kraftaufwand verringert, der erforderlich ist, um den Ballonkörper in den länglichen Kanal der länglichen medizinischen Vorrichtung zurückzuziehen, nachdem der Ballonkörper sowohl aufgeblasen als auch entleert wurde, im Vergleich zu einem identischen Ballon, dem die Gleitmittelschicht fehlt.

10. Anordnung nach Anspruch 9, wobei der Ballon im aufgeblasenen Zustand einen Durchmesser von mehr als 12 mm umfasst und der längliche Kanal der länglichen medizinischen Vorrichtung einen Durchmesser von 3,8 mm oder weniger umfasst, und wobei das Verhältnis des Durchmessers des Ballons im aufgeblasenen Zustand zum Durchmesser des länglichen Kanals größer als 4 ist.

11. Verfahren zum Herstellen eines Ballons zum Einsatz in einer Höhle eines Patienten, wobei das Verfahren Folgendes umfasst:
Erhalten eines Ballonkörpers, umfassend Polyetherblockamid (PEBAX) oder Nylon;
Aufbringen einer Polyvinylpyrrolidon-Beschichtung auf eine innere Oberfläche des Ballonkörpers; und Aushärten der Beschichtung;
wobei die ausgehärtete Beschichtung ein permanentes Gleitmittel auf der inneren Oberfläche des Ballonkörpers bereitstellt.

12. Verfahren nach Anspruch 11, wobei das Aufbringen der Beschichtung mindestens ein teilweises Füllen des Ballonkörpers mit einer Mischung umfasst, die Polyvinylpyrrolidon und einen Katalysator umfasst, und wobei das Aushärten der Beschichtung das Aussetzen der Beschichtung gegenüber UV-Licht umfasst.

## Revendications

1. Ballonnet destiné à être utilisé dans des procédures médicales, le ballonnet comprenant :
un premier matériau définissant un corps de ballonnet configuré pour une délivrance à travers un canal d'un dispositif médical allongé, le premier matériau comprenant un polyéther bloc amide (PEBAX) ou du nylon, dans lequel le corps de ballonnet comprend un diamètre, lorsqu'il est gonflé, de 10 mm ou plus ; et
un deuxième matériau disposé sur une surface interne du corps de ballonnet, le deuxième matériau comprenant une couche de lubrifiant solide séchée qui est hydrophile et comprend de la polyvinylpyrrolidone, dans lequel la couche de lubrifiant est durcie et liée de manière permanente à la surface interne du corps de ballonnet ; et
dans lequel la couche de lubrifiant est configurée pour diminuer la quantité de force nécessaire pour retirer le corps de ballonnet dans le canal du dispositif médical allongé après que le corps de ballonnet a été gonflé et dégonflé par rapport à un ballonnet identique qui est dépourvu de la couche de lubrifiant.

2. Ballonnet destiné à être utilisé selon la revendication 1, dans lequel la couche de lubrifiant est configurée pour faciliter le compactage du corps de ballonnet lorsque le corps de ballonnet est retiré dans un canal d'un dispositif médical allongé.

3. Ballonnet destiné à être utilisé selon la revendication 1, dans lequel la couche de lubrifiant est configurée pour retenir de l'eau après avoir été gonflée avec une solution aqueuse.

4. Ballon destiné à être utilisé selon l'une quelconque des revendications 1 à 3, dans lequel le corps de ballonnet comprend du nylon.

5. Ballonnet destiné à être utilisé selon l'une quelconque des revendications 1 à 3, dans lequel le corps de ballonnet comprend un polyéther bloc amide (PEBAX).

6. Ballonnet destiné à être utilisé selon l'une quelconque des revendications 1 à 5, dans lequel le corps de ballonnet, lorsqu'il est gonflé, comprend une surface extérieure telle qu'une ligne tangente à la surface extérieure coupe un axe longitudinal du corps de ballonnet à un angle aigu qui est supérieur à 65 degrés.

7. Ballonnet destiné à être utilisé selon l'une quelconque des revendications 1 à 6, dans lequel le ballonnet est configuré pour dilater une endoprothèse œsophagienne.

8. Ballonnet destiné à être utilisé selon l'une quelconque des revendications 1 à 7, dans lequel le lubrifiant comprend une couche de polyvinylpyrrolidone qui a été durcie par un processus comprenant une exposition à la lumière UV.

9. Ensemble de dispositif médical comprenant :
un ballonnet destiné à être utilisé selon l'une quelconque des revendications 1 à 8, comprenant un corps de ballonnet et une couche de lubrifiant ;
et
un dispositif médical allongé comprenant un canal allongé ;
dans lequel le corps de ballonnet est configurée pour une délivrance à travers le canal allongé du dispositif médical allongé, et la couche de lubrifiant diminue la quantité de force nécessaire pour retirer le corps de ballonnet dans le canal allongé du dispositif médical allongé après que le corps de ballonnet a été gonflé et dégonflé par rapport à un ballonnet identique qui est dépourvu de la couche de lubrifiant.

10. Ensemble selon la revendication 9, dans lequel le ballonnet comprend un diamètre supérieur à 12 mm lorsqu'il est gonflé et le canal allongé du dispositif médical allongé comprend un diamètre de 3,8 mm ou moins, et dans lequel le rapport du diamètre du ballonnet lorsqu'il est gonflé au diamètre du canal allongé est supérieur à 4.

11. Procédé de fabrication d'un ballonnet destiné à être déployé au sein d'une cavité de patient, le procédé comprenant :
l'obtention d'un corps de ballon comprenant un polyéther bloc amide (PEBAX) ou du nylon ;
l'application d'un revêtement de polyvinylpyrrolidone sur une surface interne du corps de ballonnet ; et le durcissement du revêtement ;
dans lequel le revêtement durci fournit un lubrifiant permanent sur la surface interne du corps de ballonnet.

12. Procédé selon la revendication 11, dans lequel l'application du revêtement comprend le remplissage au moins partiel du corps de ballonnet avec un mélange comprenant de la polyvinylpyrrolidone et un catalyseur et dans lequel le durcissement du revêtement comprend l'exposition du revêtement à la lumière UV.
